# EUROPÄISCHE PATENTSCHRIFT

(11) **EP 0 728 176 B1**
(45) Veröffentlichungstag und Bekanntmachung des Hinweises auf die Patenterteilung: **30.09.1998**
(21) Anmeldenummer: 94931015.5
(22) Anmeldetag: 31.10.1994
(51) Int. Cl.: C11C 3/04, C11B 3/04, C07C 67/03, C07C 69/24

(54) **VERFAHREN ZUR HERSTELLUNG VON FETTSÄURENIEDRIGALKYLESTERN**
METHOD OF PRODUCING LOWER-ALKYL ESTERS OF FATTY ACIDS
PROCEDE DE FABRICATION D'ALKYLESTERS INFERIEURS D'ACIDES GRAS

(30) Priorität: 08.11.1993 DE 4338111
(43) Veröffentlichungstag der Anmeldung: 28.08.1996
(73) Patentinhaber: Henkel Kommanditgesellschaft auf Aktien, 40191 Düsseldorf (DE)
(72) Erfinder: DEMMERING, Günther, D-42653 Solingen (DE); PELZER, Christian, D-52441 Linnich (DE); FRIESENHAGEN, Lothar, D-40589 Düsseldorf (DE)
(86) Internationale Anmeldenummer: EP9403576
(87) Internationale Veröffentlichungsnummer: WO9513343

(56) Entgegenhaltungen:
- EP-A- 0 127 104
- FR-A- 978 993
- GB-A- 377 336
- US-A- 2 177 407
- US-A- 2 383 579

## Beschreibung

### Gebiet der Erfindung

Die Erfindung betrifft ein Verfahren zur Herstellung von Fettsäureniedrigalkylestern durch Umesterung von Fettsäureglyceriden mit niederen aliphatischen Alkoholen.

### Stand der Technik

Fettsäuremethylester stellen wichtige industrielle Rohstoffe für die Herstellung einer Vielzahl von Produkten, beispielsweise Schmierstoffen und Tensiden, dar. Üblicherweise geht man zur Herstellung der Ester von natürlichen Fetten und Ölen, also Voll- oder Partialestern des Glycerins mit Fettsäuren, aus, die in Gegenwart von Katalysatoren mit Methanol umgeestert werden. Verfahren, die die Umesterung von Fetten und Ölen zum Gegenstand haben, sind in einer Vielzahl von Publikationen beschrieben; stellvertretend soll an dieser Stelle auf den Übersichtsartikel in Seifen-Öle-Fette-Wachse, 114, 595 (1988) verwiesen werden.

Um die Umesterung in einem aus wirtschaftlicher Sicht rentablen Zeitraum mit befriedigenden Umsetzungsgraden durchführen zu können, müssen Katalysatoren eingesetzt werden. Hierfür kommen insbesondere Schwermetallverbindungen, wie beispielsweise Zinkoxid [**GB 71 27 47**] oder Zinksilikat [**US 2,727,049**] in Betracht. Derartige Verfahren haben jedoch den Nachteil, daß die Katalysatoren nach der Umesterung aus toxikologischen Gründen nicht im Produkt verbleiben können, sondern mit hohem technischen Aufwand abgetrennt werden müssen.

Die Umesterung kann auch in Gegenwart von Mineralsäuren durchgeführt werden, die nach Abschluß der Reaktion lediglich neutralisiert werden müssen. Auch wenn das Problem der Katalysatorabtrennung hierbei entfällt, sind die Raum-Zeit-Ausbeuten an Umesterungsprodukten im Vergleich zur Schwermetallkatalyse deutlich schlechter, was eine technische Anwendbarkeit des Verfahrens relativiert. Eine Ausbeute, die derjenigen entspricht, die man mit den oben genannten Schwermetallverbindungen erhält, erreicht man, wenn man die Umesterung in Gegenwart von freien Fettsäuren durchführt. Ein derartiges Verfahren ist in der **WO 93/1263** (Henkel) offenbart.

In den oben genannten katalytischen Umesterungsverfahren ist es notwendig, die als Ausgangsverbindungen eingesetzten natürlichen Fettsäureglyceride mit Bleicherde ("Fullern") zu behandeln, um auf diesem Wege eine Abtrennung von Schalenresten und Schleimstoffen zu erreichen. Diese Vorbehandlung ist erforderlich, um ein möglichst geruchfreies und hell gefärbtes Produkt zu erhalten. Nach Abtrennung der Bleicherde kann die Umesterungsreaktion in Gegenwart der oben beschriebenen Katalysatoren durchgeführt werden. Durch die Behandlung mit Bleicherde werden jedoch die Schalenreste und Schleimstoffe nicht vollständig abgetrennt, so daß das aus der Umesterung von Fettsäureglyceriden mit niederen aliphatischen Alkoholen erhaltene Produkt in der Regel mit einem unangenehmen Geruch behaftet ist und verfärbt ist.

Die Aufgabe der Erfindung bestand somit darin, ein wirtschaftlich rentables Verfahren zur Umesterung von Fettsäureglyceriden zu entwickeln, das einen Fettsäureniedrigalkylester frei von unangenehmen Gerüchen und ohne Verfärbungen zur Verfügung stellt.

### Beschreibung der Erfindung

Gegenstand der Erfindung ist ein Verfahren zur Herstellung von Fettsäureniedrigalkylestern, worin man Voll- und/oder Partialester des Glycerins mit Fettsäuren der Formel **(I)**,

**R**^{**1**}**-COOH** **(I)**

in der R¹ für einen aliphatischen Kohlenwasserstoffrest mit 5 bis 23 Kohlenstoffatomen und 0 oder 1 bis 5 Doppelbindungen steht, bei erhöhter Temperatur und gegebenenfalls unter erhöhtem Druck mit niederen aliphatischen Alkoholen mit 1 bis 4 Kohlenstoffatomen umsetzt, welches dadurch gekennzeichnet ist, daß man die Voll- und/oder Partialester des Glycerins mit Fettsäuren mit der Formel (I) vor der Umesterung bei einer Temperatur von 75°C bis 120°C mit 0,3 bis 3 Gew.-%, bezogen auf die Menge der Voll- und/oder Partialester des Glycerins mit Fettsäuren mit der Formel (I), mit einer Säure behandelt.

Überraschenderweise wurde gefunden, daß die Umesterung von Fettsäureglyceriden in guten Ausbeuten und unter Bildung von geruchsfreien und nicht verfärbten Produkten erreicht werden kann, wenn man die eingesetzten Voll- und/oder Partialester des Glycerins mit Fettsäuren vor der Umesterungsreaktion mit einer Säure behandelt. Dieses Verfahren hat den Vorteil, daß 1. die in den natürlichen Ausgangsprodukten vorhandenen Schalenreste und Schleimstoffe vollständig entfernt werden, wodurch ein weißes, geruchfreies Produkt erhalten wird, und 2. durch die Behandlung mit Säure bereits eine Säurezahl erreicht ist, bei welcher die Umesterungsreaktion durchgeführt werden kann, so daß auf den Zusatz eines weiteren Katalysators verzichtet werden kann.

Als Einsatzstoffe für die Umesterung kommen im Sinne des erfindungsgemäßen Verfahrens sowohl Voll- als auch Partialester des Glycerins mit Fettsäuren in Betracht; bevorzugt ist der Einsatz von Triglyceriden. Typische Beispiele sind Glycerinester der Capronsäure, Caprylsäure, Caprinsäure, Laurinsäure, Myristylsäure, Palmitinsäure, Palmitoleinsäure, Stearinsäure, Ölsäure, Elaidinsäure, Petroselinsäure, Linolsäure, Linolensäure, Chaulmoograsäure, Rhizinolsäure, Arachinsäure, Gadoleinsäure, Behensäure, Erucasäure, Arachidonsäure und Clupanodonsäure.

Bei den Glycerinestern der genannten Fettsäuren handelt es sich um natürliche Produkte, insbesondere auch um solche, bei denen das Glycerin mit zwei oder drei verschiedenen Fettsäuren verknüpft ist. Unter Glycerinfettsäureestern auf natürlicher Basis sind Fette und Öle tierischer oder pflanzlicher Herkunft zu verstehen, beispielsweise Kokosöl, Palmöl, Palmkernöl, Erdnußöl, Baumwollsaatöl, Rapsöl, Sonnenblumenöl, Korianderöl, Leinöl, Sojaöl, Rindertalg oder Fischöl.

Erfindungsgemäß werden die eingesetzten Voll- und/oder Partialester des Glycerins vor der Umesterungsreaktion mit 0,3 bis 3 Gew.-%, bevorzugt 0,8 bis 1,5 Gew.-%, bezogen auf die Menge der Voll- und/oder Partialester des Glycerins, einer Säure behandelt. Als Säuren kommen H₂SO₄, Mineralsäuren und H₃PO₄ in Betracht. Bevorzugt wird H₂SO₄ eingesetzt. Die Säurebehandlung wird bei einer Temperatur von 75°C bis 120°C, bevorzugt 80°C bis 98°C, durchgeführt. Man läßt die Säure über einen Zeitraum von 3 bis 36 Stunden, bevorzugt 8 bis 16 Stunden, auf die eingesetzten Voll- und/oder Partialester des Glycerins einwirken. Anschließend muß die Säure vollständig abgetrennt werden, z. B. durch Waschen mit Wasser, Extraktion oder Zentrifugieren. Die eingesetzte Säure sollte möglichst vollständig entfernt werden, um Nebenreaktionen, wie die Bildung von Sulfoestern, zu vermeiden. Die so behandelten Voll- und/oder Partialester des Glycerins mit Fettsäuren mit der Formel (I) weisen nach der Säurebehandlung und vor der Umesterung eine Säurezahl von 20 bis 70, bevorzugt von 35 bis 70, auf. Es ist überraschend, daß die Umesterungsreaktion auch bei hohen Säurezahlen bis zur Säurezahl 70 problemlos durchgeführt werden kann, ohne daß bei der Umesterung gleichzeitig die Rückreaktion in Säure und Alkohol stattfindet. Auf die Zugabe von zusätzlichen Katalysatoren kann verzichtet werden.

Als niedere aliphatische Alkohole, die gegen das Glycerin im Fettsäureester ausgetauscht werden, kommen Ethanol, n-Propylalkohol, i-Propylalkohol, n-Butanol oder tert.-Butanol in Betracht. Bevorzugt wird die Umesterung mit Methanol durchgeführt.

Die Umesterung des vorbehandelten Glycerinesters kann in an sich bekannter Weise bei erhöhter Temperatur und gegebenenfalls unter erhöhtem Druck, beispielsweise bei Temperaturen von 150°C bis 300°C, insbesondere 200°C bis 250°C und Drücken von 1 bis 100 bar, insbesondere 50 bis 80 bar durchgeführt werden. An die Umesterung kann sich eine Aufarbeitung anschließen, bei der das freigesetzte Glycerin und Reste nicht umgesetzten Alkohols abgetrennt werden und der erhaltene Fettsäureniedrigalkylester destilliert bzw. fraktioniert wird.

Die folgenden Beispiele sollen den Gegenstand der Erfindung näher erläutern, ohne ihn darauf einzuschränken.

### Beispiele

In einem Rührreaktor wurden 320 g (0,5 Mol) unraffiniertes Kokosöl der Fettsäurezusammensetzung

| | |
|---|---|
| Capronsäure | 0,5 Gew.-% |
| Caprylsäure | 8 Gew.-% |
| Caprinsäure | 7 Gew.-% |
| Laurinsäure | 48 Gew.-% |
| Myristinsäure | 17 Gew.-% |
| Palmitinsäure | 9 Gew.-% |
| Stearinsäure | 2 Gew.-% |
| Ölsäure | 7 Gew.-% |
| Linolsäure | 1,5 Gew.-% |

| | |
|---|---|
| Gebundenes Glycerin | 13,8 Gew.-% |
| Säurezahl | 9,3 |

mit 50 %iger Schwefelsäure im Massenverhältnis 99:1, 24 Stunden bei 95°C unter ständigem Rühren behandelt. Die eingesetzte Schwefelsäure wurde durch Extraktion entfernt. Nach der Behandlung mit Schwefelsäure wurden für das Kokosöl die folgenden Kennzahlen bestimmt:

| | |
|---|---|
| gebundenes Glycerin | 13,1 |
| Säurezahl | 67 |

Das so vorbehandelte Kokosöl wurde in einem 1-1-Autoklaven mit 320 g (10 Mol) Methanol (Volumenverhältnis 1:1) vorgelegt. Anschließend wurde die Reaktionsmischung über einen Zeitraum von 180 Minuten bei einer Temperatur von 240°C und einem Druck von 65 bar gehalten. Nach dem Abkühlen und Entspannen der Reaktionsmischung wurde ein Umesterungsprodukt erhalten, das die folgenden Kennzahlen aufwies:

| | |
|---|---|
| gebundenes Glycerin | 1,1 Gew.-% |
| Säurezahl | 2,3. |

## Patentansprüche

1. Verfahren zur Herstellung von Fettsäureniedrigalkylestern, worin man Voll- und/oder Partialester des Glycerins mit Fettsäuren der Formel **(I)**,
**R**^{**1**}**-COOH** **(I)**
in der R¹ für einen aliphatischen Kohlenwasserstoffrest mit 5 bis 23 Kohlenstoffatomen und 0 oder 1 bis 5 Doppelbindungen steht, bei erhöhter Temperatur und gegebenenfalls unter erhöhtem Druck mit niederen aliphatischen Alkoholen mit 1 bis 4 Kohlenstoffatomen umsetzt, **dadurch gekennzeichnet,** daß man die Voll- und/oder Partialester des Glycerins mit Fettsäuren mit der Formel (I) vor der Umesterung bei einer Temperatur von 75°C bis 120°C mit 0,3 bis 3 Gew.-%, bezogen auf die Menge der Voll- und/oder Partialester des Glycerins mit Fettsäuren mit der Formel (I), mit einer Säure behandelt.

2. Verfahren nach Anspruch 1, **dadurch gekennzeichnet,** daß als Säure H₂SO₄, Mineralsäuren, H₃PO₄ eingesetzt wird.

3. Verfahren nach Anspruch 1, **dadurch gekennzeichnet,** daß die Säure in einer Menge von 0,8 bis 1,5 Gew.-%, bezogen auf die Menge der Voll- und/oder Partialester des Glycerins mit Fettsäuren mit der Formel (I), eingesetzt wird.

4. Verfahren nach Anspruch 1, **dadurch gekennzeichnet,** daß die Säurebehandlung bei einer Temperatur von 80°C bis 98°C durchgeführt wird.

5. Verfahren nach Anspruch 1, **dadurch gekennzeichnet,** daß die Voll- und/oder Partialester des Glycerins mit Fettsäuren mit der Formel (I) nach der Säurebehandlung und vor der Umesterung eine Säurezahl von 20 bis 70, bevorzugt von 35 bis 70, aufweist.

6. Verfahren nach Anspruch 1, **dadurch gekennzeichnet,** daß man als niederen aliphatischen Alkohol Methanol einsetzt.

7. Verfahren nach Anspruch 1, **dadurch gekennzeichnet,** daß man Triglyceride der Fettsäuren mit der Formel (I) einsetzt.

## Claims

1. A process for the production of fatty acid lower alkyl esters, in which full and/or partial esters of glycerol with fatty acids corresponding to formula **(I)**:
**R**^{**1**}**-COOH** **(I)**
in which R¹ is an aliphatic hydrocarbon radical containing 5 to 23 carbon atoms and 0 or 1 to 5 double bonds, are reacted with lower aliphatic alcohols containing 1 to 4 carbon atoms at elevated temperature and optionally under elevated pressure, characterized in that, before the transesterification reaction, the full and/or partial esters of glycerol with fatty acids corresponding to formula (I) are treated with 0.3 to 3% by weight, based on the quantity of the full and/or partial esters of glycerol with fatty acids corresponding to formula (I), of an acid at a temperature of 75°C to 120°C.

2. A process as claimed in claim 1, characterized in that the acid used is selected from H₂SO₄, mineral acids and H₃PO₄.

3. A process as claimed in claim 1, characterized in that the acid is used in a quantity of 0.8 to 1.5% by weight, based on the quantity of the full and/or partial esters of glycerol with fatty acids corresponding to formula (I).

4. A process as claimed in claim 1, characterized in that the acid treatment is carried out at a temperature of 80°C to 98°C.

5. A process as claimed in claim 1, characterized in that the full and/or partial esters of glycerol with fatty acids corresponding to formula (I) have an acid value of 20 to 70 and preferably 35 to 70 after the acid treatment and before the transesterification.

6. A process as claimed in claim 1, characterized in that methanol is used as the lower aliphatic alcohol.

7. A process as claimed in claim 1, characterized in that triglycerides of the fatty acids corresponding to formula (I) are used.

## Revendications

1. Procédé pour la production d'esters d'alkyle d'acides gras dans lequel on fait réagir des esters complets et/ou partiels du glycérol avec des acides gras de formule (I) :
R¹-COOH (I)
dans laquelle R¹ représente un reste hydrocarboné aliphatique ayant de 5 à 23 atomes de carbone et zéro ou de 1 à 5 doubles liaisons,
à température accrue et éventuellement sous pression accrue, avec des alcools aliphatiques ayant de 1 à 4 atomes de carbone,
caractérisé en ce qu'
on traite les esters complets et/ou partiels du glycérol avec des acides gras, ayant la formule (I), préalablement à la transestérification, à une température de 75°C à 120°C avec 0,3 à 3 % en poids, rapporté à la quantité d'esters complets et/ou partiels du glycérol d'acides gras ayant la formule (I) avec un acide.

2. Procédé selon la revendication 1,
caractérisé en ce que
comme acide, on met en oeuvre SO₄H₂, des acides minéraux et PO₄H₃.

3. Procédé selon la revendication 1,
caractérisé en ce que
l'acide est mis en oeuvre dans une quantité allant de 0,8 à 1,5 % en poids, rapporté à la quantité d'esters complets et/ou partiels du glycérol avec des acides gras ayant la formule (I).

4. Procédé selon la revendication 1,
caractérisé en ce qu'
on effectue le traitement par un acide à une température allant de 80°C à 98°C.

5. Procédé selon la revendication 1,
caractérisé en ce que
les esters complets et/ou partiels du glycérol avec des acides gras ayant la formule (I) possèdent après le traitement par un acide et préalablement à la transestérification, un indice d'acidité de 20 à 70, de préférence de 35 à 70.

6. Procédé selon la revendication 1,
caractérisé en ce qu'
on met en oeuvre comme alcool aliphatique inférieur, du méthanol.

7. Procédé selon la revendication 1,
caractérisé en ce qu'
on met en oeuvre des triglycérides des acides gras, ayant la formule (I).
